(19) Europäisches Patentamt
European Patent Office
Office européen des brevets

(11) Veröffentlichungsnummer: **0 017 206**
**B1**

(12) # EUROPÄISCHE PATENTSCHRIFT

(45) Veröffentlichungstag der Patentschrift: **02.06.82**

(51) Int. Cl.³: **C 07 C 47/04**

(21) Anmeldenummer: **80101701.3**

(22) Anmeldetag: **29.03.80**

(54) **Verfahren zur gleichzeitigen Herstellung von konzentrierten, wässrigen Formaldehyd/Harnstoff-Lösungen und Formaldehydlösungen.**

(30) Priorität: **07.04.79 DE 2914135**

(43) Veröffentlichungstag der Anmeldung: **15.10.80 Patentblatt 80/21**

(45) Bekanntmachung des Hinweises auf die Patenterteilung: **02.06.82 Patentblatt 82/22**

(84) Benannte Vertragsstaaten: **BE CH DE FR GB NL**

(56) Entgegenhaltungen:
**DE-A-2 451 990**
**GB-A-1 235 138**

(73) Patentinhaber: **BASF Aktiengesellschaft, Carl-Bosch-Strasse 38, D-6700 Ludwigshafen (DE)**

(72) Erfinder: **Aicher, Albrecht, Sonnenstrasse 21, D-6710 Frankenthal (DE)**
Erfinder: **Dudeck, Christian, Dr., Odenwaldring 20, D-6703 Limburgerhof (DE)**
Erfinder: **Petri, Norbert, Dr., Max-Beckmann-Strasse 17, D-6710 Frankenthal (DE)**
Erfinder: **Sebastian, Robert, Katharinenstrasse 4, D-6705 Deidesheim (DE)**
Erfinder: **Schuchart, Peter, Dr., Bensheimer Ring 13 B, D-6710 Frankenthal (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

# Verfahren zur gleichzeitigen Herstellung von konzentrierten, wäßrigen Formaldehyd/Harnstoff-Lösungen und Formaldehydlösungen

Die Erfindung betrifft ein Verfahren zur gleichzeitigen Herstellung von konzentrierten, wäßrigen Formaldehydlösungen und Formaldehyd/Harnstoff-Lösungen durch oxidierende Dehydrierung von Methanol mit Luft in Gegenwart eines Silberkatalysators bei erhöhter Temperatur und anschließender Absorption des gebildeten Formaldehyds in wäßriger Formaldehyd/Harnstofflösung bei einem pH unterhalb 7.

Bei der technischen Herstellung von Formaldehyd aus Methanol durch dehydrierende Oxidation mit Luft an Silberkatalysatoren in Gegenwart von Wasserdampf wird üblicherweise der Formaldehyd aus den Reaktionsgasen mit Wasser ausgewaschen. Wenn der Formaldehyd, wie es in großem Ausmaße geschieht, zur Herstellung von Polykondensationsharzen, z. B. von Harnstoff-Formaldehydharzen, verwendet wird, müssen derartige Harzlösungen eingeengt werden, wobei erhebliche Mengen Wasser zu verdampfen sind. Um das Einengen der Harzlösungen, das zeitraubend ist, einen großen Energieaufwand verlangt und mit Formaldehydverlusten verbunden ist, zu vermeiden, hat man schon versucht, hochkonzentrierte Formaldehydlösungen durch Absorption der Formaldehydgase in sauren oder basischen Harnstofflösungen herzustellen. Sowohl das saure als auch das basische Verfahren haben gewisse Nachteile. So scheidet sich bei der Absorption des gasförmigen Formaldehyds im sauren Bereich, insbesondere bei Abwesenheit von restlichem Methanol, leicht unlöslicher Methylenharnstoff ab. Im alkalischen Bereich wiederum scheiden sich leicht unlösliche Methylolharnstoffe ab, die zur Verstopfung der Absorptionstürme führen können. Einen besseren Weg zeigen Verfahren, bei denen die Absorption des Formaldehyds in Lösungen von Formaldehyd und Harnstoff, wo beide Komponenten schon bis zu einem gewissen Grad miteinander reagiert haben (Ankondensation), z. B. in Lösungen entsprechender Vorkondensate, erfolgt. Als Vorkondensate bezeichnet man in diesem Zusammenhang niedermolekulare Verbindungen von Harnstoff und Formaldehyd, in der Hauptsache ein Gemisch von Methylolharnstoff, Dimethylolharnstoff, Trimethylolharnstoff, Tetramethylolharnstoff und daneben Methylendiharnstoff, Dimethylentriharnstoff, Trimethylentetraharnstoff; ebenfalls sind in solchen Vorkondensatgemischen auch unsubstituierter Harnstoff, freier Formaldehyd, niedermolekulare Polymethylolverbindungen der vorgenannten Methylenharnstoffe, Methylenätherverbindungen mit Brückenglieder

$$-NH-CH_2-O-CH_2-NH-$$

oder verzweigte oder vernetzte, niedermolekulare Methylenharnstoffe, z. B. mit der Gruppe

$$-NH-CO-NH-CH_2-\underset{\displaystyle CH_2-}{\underset{|}{N}}-CO-NH-$$

vorhanden.

Es ist aus der deutschen Patentschrift 1 168 882 bekannt, Formaldehyd aus dem durch dehydrierende Oxidation von Methylalkohol in Gegenwart eines Silberkatalysators stammenden Gas zu absorbieren, indem man als Absorptionsflüssigkeit wäßrige Lösungen von Harnstoff-Formaldehyd-Vorkondensaten mit einem Molverhältnis von Harnstoff zu Formaldehyd von 1 : 1,8 bis etwa 1 : 3 verwendet und während der Absorption einen pH-Wert von 2,5 bis 7 aufrechterhält. Nur ein Teil des in die Lösung eintretenden Formaldehyds wird absorbiert, der Rest entweicht während der Absorption wieder in das Dampf/Gas-Gemisch und gelangt so in das Abgas; wie auch Beispiel 1 zeigt, können auf diese Weise nur Lösungen mit bis zu 40 bis 41 Gewichtsprozent Formaldehyd erhalten werden.

Aus der britischen Patentschrift 1 235 138 ist es bekannt, Formaldehyd durch Umsetzung von Methanol in der Gasphase mit Sauerstoff enthaltenden Gasen bei Temperaturen von 600 bis 800°C an Silberkatalysatoren herzustellen und das Formaldehyd enthaltende Gas in mindestens 3 Stufen bei 60 bis 90°C zu absorbieren, wobei man das Gas in der ersten bis zur vorletzten Stufe mit wäßrigen, sauren Lösungen von Harnstoff-Formaldehyd-Vorkondensaten und in der letzten Stufe mit einer wäßrigen Harnstofflösung in Berührung bringt.

Bei allen diesen Silber als Katalysator verwendenden Verfahren fallen im allgemeinen Lösungen von Formaldehyd und Harnstoff an, die 35 bis 42 Gewichtsprozent Formaldehyd enthalten. Für die Weiterverarbeitung sind aber Lösungen mit einem größeren Formaldehydanteil erwünscht. Die bei diesen Verfahren übliche Arbeitsweise, durch Einengen der Absorptionslösungen hochkonzentrierte Lösungen zu erhalten, ist im Hinblick auf Wirtschaftlichkeit und einfache Betriebsweise unbefriedigend.

Es ist aus der deutschen Offenlegungsschrift 2 224 258 bekannt, die Absorption des gebildeten Formaldehyds in einer wäßrigen Lösung von Formaldehyd und Harnstoff bei einem pH-Wert unterhalb 7 durchzuführen und die so erhaltenen Lösungen mit Luft, die anschließend für die Dehydrierung verwendet wird, zu behandeln. In einer bevorzugten Ausführungsform läßt man das gasförmige Reaktionsgemisch nacheinander mindestens 3 Absorptionskolonnen durchlaufen, wobei man es in der ersten bis zur vorletzten Stufe mit wäßrigen Lösungen von Harnstoff-Formaldehyd-Vorkondensaten und in der letzten Stufe mit einer wäßrigen Harnstofflösung in Berührung bringt. Vorzugsweise verwendet man insgesamt 4 oder allenfalls 5 hintereinandergeschaltete Absorptionsstufen. In der vorletzten bis zur ersten Absorptionsstufe wird als Absorptionsflüssigkeit eine wäßrige Lösung eines Harnstoff-Formaldehyd-Vorkondensates verwendet, welches aus einer wäßrigen Harnstoff-Formaldehyd-Lösung mit einem Molverhältnis Formaldehyd zu Harnstoff von mindestens 1,8 bis etwa 2,8, vorzugsweise von 2 bis 2,4 zu 1, durch Kondensation im sauren Bereich, beispielsweise unter Zugabe von Ameisensäure, bei einem pH-Wert von vorzugsweise 4 bis 6,5, insbesondere 5 bis 6, und einer Temperatur von 60 bis 95°C, vorzugsweise von 85 bis 95°C, in an sich bekannter Weise hergestellt wird. Die Kondensation wird dabei so weit geführt, daß in den Lösungen etwa 7 bis 25 Gewichtsprozent des Formaldehyds in Form von Methylenbrücken und 30 bis 60 Gewichtsprozent in Form von Methylolharnstoffen gebunden sind. In den Lösungen wird anschließend durch weitgehende Neutralisation, z. B. durch Zugabe von Natronlauge, ein pH-Wert von unterhalb 7, zweckmäßig 6 bis 7, eingestellt. Die aus der ersten bis vorletzten Stufe abgezogenen Lösungen haben im allgemeinen pH-Werte zwischen 4 und 7, vorzugsweise 5 bis 6 in der 1., 5,5 bis 6,5 in der 2., 6 bis 6,9 in der 3., 7,5 bis 9 in der 4. Stufe. Die bevorzugte und im Ausführungsbeispiel veranschaulichte Ausführungsform des Verfahrens besteht darin, daß man die aus der letzten Absorptionsstufe erhaltene wäßrige Formaldehyd-Harnstoff-Lösung zur Herstellung der wäßrigen Lösung des Formaldehyd-Harnstoff-Vorkondensates verwendet. Man arbeitet so, daß man die aus der letzten Stufe erhaltene Lösung mit so viel der aus der ersten Absorptionsstufe abgezogenen konzentrierten Formaldehydlösung mischt, daß die Mischung je Mol Harnstoff 1,8 bis etwa 2,8, vorzugsweise 2 bis 2,4 Mol Formaldehyd enthält. Die Mischung wird anschließend kondensiert, neutralisiert und schließlich in den der letzten Stufe vorgeschalteten Absorptionsstufen als Absorptionsflüssigkeit bei einem pH-Wert unterhalb 7 verwendet. Lösungen von im allgemeinen 47 bis 56 Gewichtsprozent absorbiertes Formaldehyd werden erhalten.

Es ist aus der deutschen Offenlegungsschrift 2 451 990 ein weiteres Verfahren der Herstellung konzentrierter, wäßriger Lösungen von Formaldehyd und Harnstoff durch oxidierende Dehydrierung von Methanol mit Luft in Gegenwart eines Silberkatalysators und anschließende Absorption des so gebildeten Formaldehyd in einer wäßrigen Lösung von Formaldehyd und Harnstoff bekannt, wobei man diese so erhaltene Lösung mit Luft, die anschließend für die Dehydrierung verwendet wird, behandelt. Die Absorption wird hierbei in einer ersten Stufe in einer wäßrigen Lösung von Formaldehyd und Harnstoff mit einem Molverhältnis von 2,3 bis 5 Mol Formaldehyd je 1 Mol Harnstoff, bei einem pH-Wert zwischen 5 und 7, dann in einer zweiten Stufe mit einer wäßrigen Lösung von 25 bis 32 Gewichtsprozent Harnstoff, von 4 bis 10 Gewichtsprozent freiem Formaldehyd und von 15 bis 25 Gewichtsprozent Methylolharnstoffverbindungen bei einem pH-Wert von 7 bis 8 und in einer dritten Stufe in einer wäßrigen Lösung von Formaldehyd und Harnstoff mit einem Molverhältnis von 0,05 bis 0,4 Mol Formaldehyd je 1 Mol Harnstoff bei einem pH-Wert von 8 bis 9 durchgeführt. In einer bevorzugten Ausführungsform läßt man das gasförmige Reaktionsgemisch nacheinander mindestens 3 Absorptionskolonnen bei Temperaturen von 60 bis 90°C, insbesondere 70 bis 85°C, durchlaufen, wobei man es in der letzten Kolonne mit einer wäßrigen Harnstofflösung, in der vorletzten Kolonne mit einer wäßrigen Lösung von Harnstoff, Formaldehyd und Methylolverbindungen und in allen übrigen Kolonnen mit einer Formaldehyd-Harnstoff-Lösung in Berührung bringt. Wie die deutsche Offenlegungsschrift 2 451 990 im Hinblick auf den vorgenannten Stand der Technik unterstreicht, geht das Verfahren von der Beobachtung aus, daß eine dreistufige Arbeitsweise mit sowohl einem sauren wie auch zwei alkalischen Absorptionsmedien und einer speziellen Zusammensetzung der 3 Medien mit Bezug auf die Menge an Harnstoff, freiem Formaldehyd und Methylolharnstoffverbindungen die Herstellung hochkonzentrierter, stabiler, wäßriger Lösungen erzielt. Lösungen von im allgemeinen 45 bis 60 Gewichtsprozent absorbiertem Formaldehyd werden erhalten. Es wird darauf hingewiesen, daß die Luftbehandlung wesentlich ist, da mit der Luft Anteile von unumgesetztem Methanol sowie Wasser und nicht absorbiertem, lediglich in der Lösung mitgeschlepptem Formaldehyd wieder im Kreislauf der Reaktion zugeführt werden.

Es ist aus der deutschen Offenlegungsschrift 2 444 586 bekannt, daß man konzentrierte, wäßrige Lösungen von Formaldehyd erhält, wenn man mit einer Belastung von 1000 bis 3000 kg Methanol je Quadratmeter Katalysatorbettquerschnitt und Stunde und in Gegenwart einer Menge von 0,1 bis 1,8 Mol Wasser und von 0,04 bis 0,4 Mol Formaldehyd je Mol dem Katalysator insgesamt zugeführtem Methanol umsetzt, wobei der Formaldehyd und von 0,1 bis 1,8 Mol Wasser je Mol dem Katalysator insgesamt zugeführtem Methanol dem Reaktionsgemisch nach der Umsetzung entnommen und dem Ausgangsgemisch zugegeben werden. Man kann z. B. der bei der Absorption gebildeten Formaldehydlösung einen Anteil an Formaldehyd und Wasser durch die der Reaktion zugeführten Luft bei einer Temperatur von 60 bis 90°C entziehen oder einen Teil der bei der Absorption gebildeten Formaldehydlösung verdampfen und der Reaktion wieder zuführen oder beide Verfahrensweisen kombinieren. In der Regel wird die Absorption in verschiedenen Stufen, z. B. in 2 bis 4 hintereinandergeschalteten Absorptionskolonnen durchgeführt. Die Absorption wird bevorzugt in der ersten Stufe bei einer Temperatur von 55 bis 90°C und in der

zweiten Stufe von 40 bis 65° C durchgeführt. In der 3. und 4. Kolonne verwendet man im allgemeinen Temperaturen von 20 bis 65° C.

Es ist aus der deutschen Auslegeschrift 2 208 369 ein Verfahren zur kontinuierlichen Herstellung von Harnstoff-Formaldehyd-Additionsprodukten durch Einleiten von gasförmigem Formaldehyd oder Formaldehyd enthaltenden Gasen in wäßrige alkalische Harnstofflösungen bekannt, wobei man den Formaldehyd in dem unteren Teil einer einzigen Kolonne mit Hilfe einer im Kreislauf geführten wäßrigen alkalisch gehaltenen und ständig durch Harnstoff ergänzten Harnstoff-Formaldehyd-Lösung absorbiert, dann den nicht absorbierten Formaldehyd in dem oberen Teil der Kolonne mit Wasser auswäscht und die gebildete wäßrige Formaldehydlösung unmittelbar in den unteren Teil der Kolonne überführt und vorteilhaft aus dem oberen Teil der Kolonne eine größere Menge Wasser in Form von Wasserdampf abzieht als Wasser zur Auswaschung des Formaldehyds auf die Kolonne aufgegeben wird. Bevorzugt hält man die Temperatur im unteren Teil der Kolonne auf etwa 50 bis 85° C und im oberen Teil der Kolonne auf etwa 20 bis 50° C. Die Beispiele zeigen pH-Werte von 8,1 bis 8,2.

Es ist aus der deutschen Auslegeschrift 1 239 290 bekannt, daß man Formaldehyd in einer mehrstufigen Absorptionsapparatur im Gegenstrom zu einer wäßrigen Harnstofflösung bei 30 bis 80° C und bei einem pH-Wert von 7 bis 9, vorzugsweise 8,2, leitet, dann den in dieser Stufe nicht absorbierten gasförmigen Formaldehyd in den nachfolgenden Stufen in Wasser absorbiert. Wie Beispiel 4 zeigt, beträgt die Absorptionstemperatur in der ersten Kolonne 40° C, der pH-Wert in der ersten Kolonne 7,5 und die Absorptionstemperatur in der zweiten Kolonne 20° C. Man erhält eine Formaldehyd/Harnstofflösung mit einem Formaldehydgehalt von 49 Prozent und eine 12gewichtsprozentige Formaldehydlösung. Alle diese Verfahren befriedigen im Hinblick auf Gesamtausbeute und Raum-Zeit-Ausbeute an verwertbarem Formaldehyd, einfachem und wirtschaftlichem Betrieb, Methanolverlust oder Formaldehydgehalt im Abgas nicht.

Es wurde nun gefunden, daß man gleichzeitig konzentrierte wäßrige Formaldehyd/Harnstoff-Lösungen und Formaldehydlösungen durch oxidierende Dehydrierung von Methanol mit Luft in Gegenwart eines Silberkatalysators bei erhöhter Temperatur und anschließender Absorption des so gebildeten Formaldehyds in einer wäßrigen Lösung von Formaldehyd und Harnstoff bei einem pH-Wert unterhalb 7 vorteilhaft erhält, wenn die Absorption

a) in einer ersten Stufe mit einer wäßrigen Lösung von 16 bis 30 Gewichtsprozent Harnstoff, von 20 bis 40 Gewichtsprozent freiem Formaldehyd, von 15 bis 40 Gewichtsprozent Methylolharnstoffverbindungen bei einem pH-Wert zwischen 2 und 7 und bei einer Temperatur von 80 bis 95° C,

b) dann in einer zweiten Stufe mit einer wäßrigen, 30- bis 45gewichtsprozentigen Formaldehydlösung bei einem pH-Wert von 2 bis 6 und bei einer Temperatur von 70 bis 85° C und

c) schließlich in einer dritten Stufe mit einer wäßrigen, 1- bis 30gewichtsprozentigen Formaldehydlösung bei einem pH-Wert von 2 bis 6 und bei einer Temperatur von 30 bis 50° C durchgeführt wird, und

d) aus der dritten Stufe kontinuierlich 0,1 bis 1,9 Mol Wasser und 0,04 bis 0,6 Mol Formaldehyd je Mol dem Katalysator insgesamt zugeführtem Methanol entnommen und dem Ausgangsgemisch der Methanoloxidation mit einer Belastung von 1000 bis 3000 kg Methanol je Quadratmeter Katalysatorbettquerschnitt und Stunde zugegeben werden.

Im Vergleich zu den bekannten Verfahren liefert das Verfahren nach der Erfindung, gerade auch im industriellen Maßstab, auf einfacherem und wirtschaftlicherem Wege bessere Gesamtergebnisse im Hinblick auf gleichzeitig hohe Konzentration der hergestellten Formaldehydlösung und der Formaldehyd/Harnstofflösung bei gleichzeitig guter Ausbeute an verwertbarem Endstoff, hohem Umsatz und niedrigem Gehalt der Lösung an Methanol und Ameisensäure. Methanol und Formaldehyd sind im Abgas nicht mehr in deutlichen Mengen enthalten. Nach dem neuen Verfahren erhält man in der Regel bei einmaligem Durchsatz des Methanols Formaldehyd-Ausbeuten von insgesamt 89 bis 91,5 Prozent der theoretisch berechneten Ausbeute, wovon 30 bis 65 Prozent in Gestalt der Formaldehyd/Harnstofflösung anfallen. Die nach der Absorption des Formaldehyds aus den formaldehydhaltigen Gasen erhaltenen wäßrigen Lösungen enthalten im allgemeinen in Stufe b) 30 bis zu etwa 45 Gewichtsprozent, vorzugsweise 38 bis 42 und in der Formaldehydlösung der Stufe c) 1 bis 30, vorzugsweise 5 bis 20 Gewichtsprozent Formaldehyd und weisen in der Formaldehyd/Harnstofflösung in der Regel lediglich einen Methanolgehalt von 0,2 bis 3, in der Formaldehydlösung der Stufe b) 0,8 bis 5 und der Stufe c) 0,8 bis 10 Gewichtsprozent auf. Der Ameisensäuregehalt der erhaltenen Formaldehydlösung ist gering und in der Regel unterhalb 0,01 Gewichtsprozent, bezogen auf eine 40gewichtsprozentige Formaldehydlösung. Das Verfahren nach der Erfindung ermöglicht gerade auch im großtechnischen Maßstab einen reibungsloseren Betrieb mit gleichzeitig hohem und konstanterem Umsatz des Ausgangsgemisches, langer Betriebsdauer des Katalysators und großem Querschnitt der Katalysatorschicht. Besondere Maßnahmen zur Verhinderung thermischer Schwankungen im Katalysator sind nicht notwendig. Diese vorteilhaften Ergebnisse werden bei gleichzeitig langer Lebensdauer des Katalysators erzielt, Schwankungen in den Ergebnissen werden somit während längerer Zeit vermieden. Eine Destillation der Formaldehydlösung ist nicht notwendig. Durch die einfachere Arbeitsweise wird weniger Formaldehyd insgesamt mit dem Abgas bzw. dem Abwasser mitgeschleppt und so die Luft- und Abwasserverschmutzung verringert; das erfindungsgemäße Verfahren ist im Hinblick auf den Stand der Technik umweltfreundlicher. Es ist ein besonderer und überraschender Vorteil des erfindungsgemäßen Verfahrens, daß höher konzentrierte, wäßrige Methanollösungen, insbesondere zwischen 78 und 100 Gewichtsprozent, bevorzugt verwendet werden können. Das erfindungsgemäße Verfahren liefert mit beiden Absorptionslösungen ohne weitere Reinigung für viele Synthesen, insbesondere auf dem Leim- und Klebstoffgebiet, sofort verwertbare Zwischenprodukte.

Im Hinblick auf die Herstellung der Formaldehyd/Harnstofflösung können überraschend und vorteilhaft mehrere, unterschiedlich zusammengesetzte Absorptionsstufen und somit entsprechende Kontroll-, Dosier- und Regelanlagen vermieden werden. Schon die eine Absorptionsstufe ermöglicht die Herstellung hochkonzentrierter, stabiler, wäßriger Lösungen. Eine Luftwäsche mit der Absorptionslösung wird eingespart. Da unumgesetztes Methanol mit der Lösung der Stufe c) wieder der Reaktion zugeführt wird, erhöht sich die Gesamtausbeute, gleichzeitig vermindert sich der Methanolgehalt im Abwasser. Die erhaltenen Formaldehyd/Harnstofflösungen lassen sich gut verarbeiten, bei der Leimherstellung verläuft z. B. die Kondensationsreaktion, die durch Methanol verzögert wird, rascher und gleichmäßiger. Die gesonderte Herstellung der Vorkondensate und damit zusätzliche Umsetzungs- und Neutralisationsoperationen werden eingespart. Die Formaldehyd/Harnstofflösungen sind salzärmer und können daher für zahlreiche Synthesen, z. B. bei der Leimherstellung, ohne Destillation direkt weiterverarbeitet werden und ergeben reinere Folgeprodukte. Die Menge an Methylenharnstoffen und Methylenäthern, die die Weiterverarbeitung zu Klebmitteln stören, ist geringer.

Diese vorteilhaften Ergebnisse sind im Hinblick auf den Stand der Technik überraschend, da ja angesichts der kurzen Verweilzeit in der ersten Absorptionsstufe bei pH-Werten unterhalb von 7 und bei relativ hoher Temperatur in der ersten und zweiten Stufe, vergleichsweise an Formaldehyd schwächer konzentrierte Absorptionslösungen, Verunreinigung der Lösungen der zweiten Stufe mit Nebenprodukten der ersten Stufe, z. B. Ammoniak, Aminen, stärkere Bildung von Nebenprodukten und somit schlechtere Gesamtausbeuten und Reinheit der verwertbaren Absorptionslösungen zu erwarten waren. Auch war zu vermuten, daß Nebenprodukte der ersten Stufe, z. B. stickstoffhaltige Verbindungen in unvorhersehbarer Weise in die Oxidation gelangen und die Aktivität des Katalysators verringern bzw. die Reaktion blokkieren.

Für die Formaldehydherstellung geeignete Ausgangsstoffe sind reines Methanol, technisches Methanol (unvollständig gereinigtes Rohmethanol, z. B. durch lediglich eine ungenügende Destillation) oder vorteilhaft deren Mischungen mit Wasser; die Konzentration der wäßrigen Gemische kann zweckmäßig zwischen 50 und 95 Gewichtsprozent, vorzugsweise zwischen 60 und 90 Gewichtsprozent Methanol schwanken. Ebenfalls kann Rohmethanol, das auch nach den in DE-AS 1 277 834, DP 1 136 318 und DP 1 235 881 beschriebenen Verfahren durch Abtrennung einer niedriger siedenden Fraktion, durch Behandlung mit Oxidationsmitteln und/oder Alkalien gereinigt sein kann, verwendet werden.

Man kann in einer bevorzugten Ausführungsform zwischen zweckmäßig 78- und 85gewichtsprozentige wäßrige Lösungen verdampfen, und das zurückgeführte Wasser und Methanol sowie den Formaldehyd diesen Lösungen im Verdampfer oder dem Dampfstrom dieser Lösungen zusetzen. Man kann beispielsweise den zurückgeführten Anteil der Formaldehydlösung gesondert verdampfen und den Dampf dann mit dem Methanoldampf und/oder mit der Luft vermischen. Bei einer anderen Variante vereinigt man den zurückgeführten Anteil der Formaldehydlösung mit weiterem Methanol und verdampft das Gesamtgemisch. Entsprechende Varianten ergeben sich durch gleichzeitige oder spätere Zumischung der Luft. Bei allen diesen Ausführungsformen kann man auch noch zusätzliche Mengen an Wasser, zweckmäßig im Gemisch mit Methanol, verwenden. Man kann den zurückgeführten Anteil der Formaldehydlösung auch dem Ausgangsgemisch im Verdampfer zufügen oder in das zugeführte, zusätzliche Wasser eindosieren oder man kann ihn zusammen mit dem Methanol in den Verdampfer einführen. Bei einer bevorzugten Ausführungsform des Verfahrens vermischt man den zurückgeführten Anteil der Formaldehydlösung mit weiterem Methanol und Wasser und verdampft das Gemisch unter gleichzeitigem Durchleiten von Luft durch die Verdampfungsflüssigkeit. In allen Fällen wird unter dem Katalysator insgesamt zugeführten Methanol stets das Gesamtgemisch des Ausgangsmethanols und des zurückgeführten Methanols verstanden.

Eine Verwendung von Inertgas ist nicht notwendig und bei Reaktionstemperaturen von mindestens 550°C und insbesondere 600°C unangebracht. Gegebenenfalls kann man mit heißen Intertgasen, zweckmäßig Stickstoff oder rußarmen Verbrennungsgasen, z. B. von einer Temperatur von 400 bis 800°C, den Katalysator erhitzen. Es kommen zweckmäßig Mengen von 0,15 bis 0,60, vorzugsweise von 0,15 bis 0,50 Mol Sauerstoff in Gestalt von Luft je Mol Methanol in Betracht.

Für das Verfahren nach der Erfindung kommen die für die Herstellung von Formaldehyd im allgemeinen verwendeten Silberkatalysatoren in Betracht, z. B. die in der deutschen Auslegeschrift 1 231 229 und Ullmanns Encyklopädie der technischen Chemie, Band 7, Seiten 659 ff.

beschriebenen. Vorzugsweise verwendet man Zwei- und Mehrschicht-Silberkatalysatoren, z. B. die in der deutschen Auslegeschrift 1 294 360 und in der deutschen Offenlegungsschrift 1 903 197 aufgeführten Katalysatoren. Bezüglich Herstellung des Katalysators und Durchführung der entsprechenden Umsetzung mit diesen Katalysatoren wird auf die genannten Veröffentlichungen verwiesen. In einer bevorzugten Ausführungsform wird die Umsetzung mit einem Katalysator mit der Gesamtschichtdicke von 15 bis 35 mm und 3 oder mehr Schichten Silberkristallen durchgeführt, wobei ein Teil der Schichten 72,5 bis 89 Gewichtsprozent des Katalysators mit Teilchen der Korngröße 1 bis 2,5 mm, ein Teil der Schichten 2,5 bis 7,5 Gewichtsprozent des Katalysators mit Teilchen der Korngröße 0,75 bis 1 mm und der restliche Teil der Schichten 8,5 bis 20 Gewichtsprozent des Katalysators mit Teilchen der Korngröße 0,1 bis 0,75, insbesondere 0,2 bis 0,75 mm enthalten. Bevorzugte Katalysatoren sind die in der deutschen Auslegeschrift 2 322 757 beschriebenen. Das Verhältnis von Durchmesser zu Dicke der Katalysatorschicht, die vorzugsweise aus zwei oder mehr Schichten unterschiedlicher Korngröße besteht, beträgt zweckmäßig mindestens 25, vorzugsweise von 40 bis 200, insbesondere von 60 bis 100. Im allgemeinen verwendet man Durchmesser von mindestens 500, vorzugsweise von 1500 bis 4000, insbesondere von 1700 bis 3000 mm.

Das dampfförmige Ausgangsgemisch enthält im allgemeinen sowohl frisch zugegebenes Wasser und Methanol (Zugabegemisch) als auch zurückgeführte Anteile von Wasser, Formaldehyd und in den meisten Fällen zurückgeführtes Methanol. Man verwendet eine Belastung von 1000 bis 3000, vorzugsweise 1400 bis 2500 kg Methanol je Quadratmeter Katalysatorbettquerschnitt und Stunde und zweckmäßig Mengen von 0,1 bis 1,9, vorzugsweise von 0,5 bis 1,5 Mol Wasser und von 0,04 bis 0,6, vorzugsweise von 0,05 bis 0,2 Mol Formaldehyd je Mol dem Katalysator insgesamt zugeführten Methanol.

Die Oxidation wird im übrigen in bekannter Weise durchgeführt, indem man z. B. das Dampf/Gasgemisch aus Methanoldampf, Wasserdampf, Formaldehyddampf, Luft und gegebenenfalls Inertgas in vorgenannten Mengen bei Temperaturen von etwa 550 bis 780° C, insbesondere 640 bis 750° C, durch den Silberkatalysator leitet. Es ist dabei zweckmäßig, die die Katalysatorzone verlassenden Reaktionsgase innerhalb kurzer Zeit, beispielsweise in weniger als 0,2 Sekunden, abzukühlen, z. B. auf Temperaturen von 100 bis 170° C. Das abgekühlte Gasgemisch wird dann zweckmäßig einem Absorptionsturm bzw. einer Kolonne der ersten Absorptionsstufe zugeführt.

Die Herstellung des Formaldehyds wird im allgemeinen bei Drücken zwischen 1 und 2 bar, vorzugsweise zwischen 1,1 und 1,8 bar, diskontinuierlich oder vorzugsweise kontinuierlich durchgeführt.

Die Absorption des Formaldehyds kann drucklos oder unter Druck, diskontinuierlich oder zweckmäßig in der Regel kontinuierlich durchgeführt werden.

Jede Absorptionsstufe kann ein oder mehrere Absorptionsgefäße, in der Regel Absorptionskolonnen umfassen: vorteilhaft enthält die erste Absorptionsstufe a) (Formaldehyd/Harnstofflösung) nur eine Kolonne, die zweite Absorptionsstufe b) (konzentrierte Formaldehydlösung) ebenfalls nur eine Kolonne und die dritte Absorptionsstufe c) (niedrig konzentrierte Formaldehydlösung) mehrere, zweckmäßig 3 und insbesondere 2 Absorptionskolonnen. Die Kolonnenlänge beträgt insgesamt bevorzugt 1 bis 15 Meter in der ersten Stufe, 1 bis 12 Meter in der zweiten Stufe, 1 bis 20 Meter in der dritten Stufe, im Falle von Bodenkolonnen bevorzugt 5 bis 15 Böden in der ersten Stufe, 5 bis 10 Böden in der zweiten Stufe, 5 bis 20 Böden in der dritten Stufe. Alle Absorptionskolonnen haben zweckmäßig einen Kreislauf der Absorptionsflüssigkeit, wobei ein Teil der Sumpfflüssigkeit über den Kopf der Kolonne im Gegenstrom zum Reaktionsgemisch geführt wird. Daneben haben die Absorptionskolonnen derselben Stufe zweckmäßig einen Flüssigkeitsrücklauf, vorteilhaft dergestalt, daß ein Teil der Sumpfflüssigkeit jeder Kolonne auf den Kopf bzw. in den Kreislauf der vorhergehenden Kolonne derselben Stufe eingeleitet wird. Ein entsprechender Rücklauf zwischen Stufe a(b) und Stufe 1(a) erfolgt nicht. Ein Rücklauf zwischen 3.(c) und 2.(b) Stufe ist möglich, erfolgt aber bevorzugt nicht. In einer vorteilhaften Ausführungsform findet nur ein Rücklauf zwischen den einzelnen Kolonnen jeder Stufe aber kein Rücklauf zwischen den einzelnen Stufen der Absorption statt. Zweckmäßig wird die Waschflüssigkeit am Kolonnenkopf möglichst gleichmäßig und rasch mit dem Reaktionsgas vermischt, z. B. über Düsen versprüht oder mittels eines perforierten Bodens verteilt. Vorteilhaft legt man in den drei Stufen anfangs soviel Wasser vor bzw. fügt stündlich soviel Wasser zu, daß entsprechende Mengen an Wasser im Gegenstrom vom Kolonnenkopf her zugeführt werden und das Wasser mit dem absorbierten Formaldehyd in der ersten, zweiten und dritten Stufe Lösungen der vorgenannten Zusammensetzungen bildet. Die zusätzlich der Absorptionsstufe zugeführte Wassermenge hängt von der Menge an kondensiertem Wasser aus dem Reaktionsgas, den jeweils aus dem Kolonnensumpf entnommenen Anteil an Lösung und gegebenenfalls vom Reaktionsgas wieder mitgeschleppten Anteilen an Wasser ab.

Die Absorption, im allgemeinen in Form einer Wäsche des Gasgemisches, wird in der Regel in Absorptionstürmen (Absorptionskolonnen) durchgeführt, z. B. wird die Absorptionsflüssigkeit am Kopf oder in der oberen Hälfte der Kolonne zugegeben und das Gasgemisch zweckmäßig im Gegenstrom von der Kolonnenblase her nach oben durch die Kolonne geleitet. Als Absorptionskolonnen kommen Siebboden-, Oldershaw-, Glasboden-, Glockenboden-, Ventil-

bodenkolonnen, Füllkörpersäulen oder Apparate mit rotierenden Einsätzen in Frage. Vorteilhaft verwendet man Bodenkolonnen, die eine Geschwindigkeit von 0,030 bis 2 Volumenteilen stündlich in die Kolonne eintretende Absorptionslösung je Volumenteil des Gesamtraumes der Kolonne erlauben. Bei Kugelventilbodenkolonnen und Siebbodenkolonnen sind Lochdurchmesser von 5 bis 15 mm, Kugeldurchmesser von 8 bis 30 mm und Bodenabstände von 300 bis 800 mm bevorzugt. Zweckmäßig sind Zulaufgeschwindigkeiten von 10 bis 70 $m^3$ Absorptionslösung pro Stunde und Quadratmeter Kolonnenquerschnitt oder ein Durchsatz von 1000 bis 6000 $m^3$ Gas pro Stunde und Quadratmeter Kolonnenquerschnitt.

Insgesamt werden in den 3 Absorptionsstufen von der wäßrigen Lösung des Harnstoff-Formaldehyd-Kondensates in der ersten Stufe a) im allgemeinen etwa 30 bis 65 Gewichtsprozent, in der zweiten Stufe b) von der wäßrigen Formaldehydlösung etwa 30 bis 50 Gewichtsprozent und in der dritten Stufe c) von der wäßrigen Formaldehydlösung etwa 5 bis 20 Gewichtsprozent der gesamten Formaldehydmenge absorbiert. Vorteilhaft betragen in der ersten Stufe a) die Verweilzeiten, bezogen auf den der Absorption zugeführten Formaldehyd aus dem Reaktionsgemisch 30 bis 240, in der zweiten Stufe b) 30 bis 240, in der dritten Stufe c) 30 bis 180 Minuten. Die pH-Werte der Absorptionslösungen sind in der ersten Stufe a) zwischen 2 und 7, vorzugsweise 4 und 6,5, in der zweiten Stufe b) von 2 bis 6, vorzugsweise von 3 bis 5, in der dritten Stufe c) von 2 bis 6, vorzugsweise von 3 bis 5. Die Absorptionstemperatur beträgt zweckmäßig in der ersten Stufe a) 80 bis 95, insbesondere 82 bis 88°C, in der zweiten Stufe b) 70 bis 85, insbesondere 72 bis 80°C, in der dritten Stufe c) 30 bis 50, insbesondere 35 bis 45°C. Die Temperatur wird zweckmäßig im Kolonnenkopf gemessen. Im allgemeinen treten insgesamt von dem nicht umgesetzten Methanol 5 bis 30 Gewichtsprozent mit der abgezogenen Lösung der ersten Absorptionsstufe a), 10 bis 40 Gewichtsprozent mit der abgezogenen Lösung der zweiten Absorptionsstufe b), 0 bis 5 Gewichtsprozent im Abgas aus, der Rest wird wieder in die Reaktion zurückgeführt.

Die vorgelegten Absorptionslösungen der ersten Absorptionsstufe a) durch Zuführung der Komponenten Formaldehyd und Harnstoff und Methylolharnstoffverbindungen auf das gewünschte Mengenverhältnis eingestellt. Unter Methylolharnstoffverbindungen werden hier Vorkondensate ohne Methylenbrücken wie die Mono- und Polymethylolharnstoffe Methylolharnstoff, Dimethylolharnstoff, Trimethylolharnstoff und Tetramethylolharnstoff, aber keine Polymethylolverbindungen von Methylenharnstoffen verstanden. Das abgekühlte gasförmige Reaktionsgemisch tritt in die erste Absorptionsstufe a), zweckmäßig eine Kolonne, ein und wird dort in einer wäßrigen Lösung von 16 bis 30, insbesondere 20 bis 25 Gewichtsprozent Harnstoff, von 20 bis 40, insbesondere 25 bis 35 Gewichtsprozent freiem Formaldehyd, von 15 bis 40, insbesondere 20 bis 30 Gewichtsprozent Methylolharnstoffverbindungen absorbiert. Neben diesen Stoffen können noch weitere Stoffe, die sich z. B. bei der Herstellung der Lösung bzw. der Absorption gebildet haben, vorhanden sein, zweckmäßig von 0,5 bis 6, vorzugsweise von 3 bis 4 Gewichtsprozent Mono- und Polymethylenharnstoffe, von 0,5 bis 3, vorzugsweise von 0,7 bis 2 Gewichtsprozent Polymethylolverbindungen von Methylenharnstoffen, von 0,1 bis 1, vorzugsweise von 0,2 bis 0,5 Gewichtsprozent Methylenätherbindungen, von 0,05 bis 0,5, vorzugsweise von 0,1 bis 0,4 Gewichtsprozent höhermolekulare Kondensate, von 0,2 bis 3, vorzugsweise von 0,4 bis 1,5 Gewichtsprozent Methanol, von 0,001 bis 0,08, vorzugsweise von 0,008 bis 0,02 Gewichtsprozent Nebenstoffe (z. B. Ameisensäure). Vorteilhaft haben solche Lösungen von 40 bis 60, vorzugsweise von 50 bis 55 Gewichtsprozent Gesamtformaldehyd und werden in diesen Absorptionsbedingungen so geregelt, daß keine Trübung zu beobachten ist. Man zieht entsprechend den absorbierten Anteilen zweckmäßig laufend Absorptionsflüssigkeit aus dem Sumpf, die die vorgenannte Zusammensetzung besitzt, ab und gibt Harnstoff in entsprechenden Anteilen nach. Bei den bevorzugten Ausführungsformen entzieht man vorteilhaft der ersten Absorptionsstufe a) (1. Kolonne) stündlich 0,5 bis 1,5 Liter Lösung je Kilogramm hergestellten und in die erste Absorptionsstufe eintretenden Formaldehyd und Stunde und führt sie der weiteren Verwendung der Lösung, z. B. der Leimherstellung, zu, und führt den Rest des Sumpfes im Kreislauf zum Kopf der Absorptionskolonne zurück.

Das Abgas aus der ersten Absorptionsstufe a) tritt nun in die zweite Absorptionsstufe b), im allgemeinen eine Absorptionskolonne (in der bevorzugten Ausführungsform die insgesamt 2. Absorptionskolonne) und wird dort in einer 30- bis 45-, insbesondere 35- bis 42gewichtsprozentigen Formaldehydlösung in vorgenannter Weise absorbiert. Durch Zusatz entsprechender Mengen Wasser wird die Lösung ständig in ihrer Zusammensetzung gehalten. Bei den bevorzugten Ausführungsformen entzieht man vorteilhaft der zweiten Absorptionsstufe b) (2. Kolonne) stündlich 0,5 bis 1,5 Liter Lösung je Kilogramm hergestellten und in die erste Absorptionsstufe eintretenden Formaldehyd und Stunde. Die bei der Absorption der zweiten Stufe b) erhaltene Formaldehydlösung enthält in der Regel von 30 bis 45, vorzugsweise von 38 bis 42 Gewichtsprozent Formaldehyd, von 0,8 bis 5, vorzugsweise von 1,5 bis 3 Gewichtsprozent Methanol und von 0,004 bis 0,025 Gewichtsprozent Ameisensäure. Das Abgas der zweiten Stufe tritt dann in die dritte Absorptionsstufe c), zweckmäßig in die erste Kolonne der dritten Absorptionsstufe c) ein.

Die zurückgeführten Stoffe (Kreislaufgemisch) Wasser, Formaldehyd und gegebenenfalls Methanol werden der Absorptionslösung in der dritten Absorptionsstufe c) entnommen. In einer bevorzugten Ausführungsform wird die erste (a) und zweite (b) Stufe jeweils mit einer Kolonne unter den vorgenannten Bedingungen betrieben. Das aus der insgesamt zweiten Kolonne (1. Kolonne der 2. Stufe) austretende Gas wird dann in die erste Kolonne der dritten Stufe c) (=3. Kolonne), zweckmäßig direkt in die im Kreislauf geführte Sumpfflüssigkeit, geleitet. Über oder neben der dritten Kolonne befinden sich die weiteren zur dritten Stufe c) gehörenden Kolonnen, z. B. eine 4. (2. Kolonne der 3. Stufe) und 5. (3. Kolonne der 3. Stufe) Absorptionskolonne. In diesen weiteren Kolonnen befindet sich ebenfalls zweckmäßig jeweils ein Kreislauf der Waschflüssigkeit wie in Kolonne 1, 2 und 3 mit zweckmäßig konstantem Pegel des Flüssigkeitssumpfes und Berieselungs- oder Sprüheinrichtungen oder Düsen am Kopf der Kolonne. Es wird nur in der 3. Stufe vorteilhaft jeweils ein entsprechender Anteil des Flüssigkeitskreislaufs einer Kolonne der vorhergehenden Kolonne zugeführt. Beispielsweise wird laufend ein Anteil der Kreislaufflüssigkeit der Kolonne 5 dem Kreislauf der Kolonne 4 am Kopf zugeführt und gleichzeitig laufend dem Sumpf der Kolonne 4 ein Anteil, der auf den Kopf der Kolonne 3 in der geschilderten Weise geleitet wird, entnommen. Die Durchsätze der nachgeschalteten Kolonnen, z. B. der 4. und 5. Kolonne, entsprechen der vorgeschalteten, in diesem Fall der 3. Kolonne (1. Kolonne der 3. Stufe c)). Im Falle einer 5-Kolonnen-Absorption mit 3 Kolonnen in der 3. Stufe c) läßt man vorteilhaft sich als Absorptionsflüssigkeiten Lösungen von 10 bis 20 Gewichtsprozent Formaldehyd (berechnet 100%) in der 3. Kolonne, von 5 bis 10 Gewichtsprozent in der 4. Kolonne und von 1 bis 3 Gewichtsprozent Formaldehyd in der 5. Kolonne einstellen, zweckmäßig durch Zugabe von Wasser, insbesondere auf den Kopf der letzten Kolonne. Bei 2 Kolonnen in der 3. Absorptionsstufe sind in der 1. (3.) Kolonne zweckmäßig Lösungen von 10 bis 20 Gewichtsprozent Formaldehyd und in der 2. (4.) Kolonne Lösungen von 1 bis 10 Gewichtsprozent Formaldehyd Absorptionsflüssigkeiten. Das aus der letzten Kolonne ausströmende Abgas enthält nur noch wenig, meist unter 0,1 Gewichtsprozent Formaldehyd und etwa 1,5 Gewichtsprozent Wasserstoff, bezogen auf das gesamte Abgas. Das Abgas ist brennbar und der Rest an Formaldehyd wird auf diese umweltfreundliche Weise verbrannt. Mit Bezug auf die gesamte Länge bzw. Bodenzahl aller Kolonnen der 3. Stufe c) ist es vorteilhaft, ein Längen- bzw. Bodenzahlverhältnis der 2. bzw. der 2. und 3. Kolonne insgesamt zur ersten Kolonne der 3. Stufe c) auf 1 bis 2 zu 1 einzustellen. Unter gesamter Länge wird die Länge des Gesamtraumes, in dem die Absorption erfolgt, verstanden.

Man entzieht der Absorptionslösung der 3. Stufe c) einen Anteil an Formaldehyd, Wasser und gegebenenfalls Methanol, nämlich von 0,04 bis 0,6, vorzugsweise von 0,08 bis 0,2 Mol Formaldehyd, von 0,1 bis 1,9, vorzugsweise von 0,8 bis 1,3 Mol Wasser und zweckmäßig von 0,001 bis 0,05, vorzugsweise von 0,01 bis 0,03 Mol Methanol je Mol dem Katalysator insgesamt zugeführten Methanol (je Mol Gesamtmethanol im Ausgangsgemisch vor der Katalysatorschicht). Vorteilhaft führt man die Absorption in vorgenannter Anlage mit 2 oder 3 Kolonnen in der 3. Stufe c) durch. Bevorzugt ist eine Ausführung mit einer Kolonne in der 1. Absorptionsstufe a), einer Kolonne in der 2. Stufe b) und 2 oder 3 Kolonnen bzw. 2 oder 3 Kolonnenaufsätzen der 3. Absorptionsstufe c), wobei aus dem Kolonnensumpf oder unterstem Boden der untersten (3.) Kolonne ein Anteil der Lösung entnommen, ein Teil der Lösung der 4. Kolonne dem Kopf der 3. Kolonne zugeführt, ein Teil der Lösung der 5. Kolonne dem Kopf der 4. Kolonne zugeführt und am Kopf der 5. Kolonne Wasser eingeleitet wird. Die letzte Kolonne ist zweckmäßig eine Bodenkolonne mit zweckmäßig 10 Böden, bei der die Absorptionslösung eine Temperatur von 30 bis 50°C aufweist. Man kann die Formaldehydlösung nur einer, zweckmäßig dem Sumpf der ersten Kolonne der 3. Stufe c), oder mehreren Kolonnen, zweckmäßig dem Sumpf der 1. (3.) und 2. (4.) Kolonne der 3. Absorptionsstufe c), entnehmen. Abgasdurchsatz und Wasserzugabe werden so eingestellt, daß vorgenannte wäßrige Formaldehydlösung der 3. Absorptionsstufe c) entnommen und zurückgeführt wird, z. B. dem Verdampfer oder einem davon getrennten 2. Verdampfer. Der 2. Verdampfer dient nur der Verdampfung der entnommenen Lösung und wird im allgemeinen unter den gleichen Bedingungen wie der vorgenannte Methanolverdampfer betrieben. Vorteilhaft führt man beide Verdampfungen in einer einzigen Kolonne mit 2 getrennten Verdampfungsstellen durch. Zweckmäßig ist ein Verhältnis von 0,03 bis 0,5, vorzugsweise von 0,05 bis 0,15 Mol aus der 1. (3.) Kolonne der 3. Stufe c) zurückgeführtem Formaldehyd, von 0,05 bis 1,5, vorzugsweise 0,5 bis 1,1 Mol aus der 1. (3.) Kolonne der 3. Stufe c) zurückgeführtem Wasser und von 0,001 bis 0,03, vorzugsweise von 0,01 bis 0,02 Mol aus der 1. (3.) Kolonne der 3. Stufe c) rückgeführtem Methanol je Mol dem Katalysator insgesamt zugeführtem Methanol (Gesamtmethanol im Ausgangsgemisch vor der Katalysatorschicht), wenn das Kreislaufgemisch aus 2 Kolonnen, in der Regel aus der 1. (3.) Kolonne und 2. (4.) Kolonne der 3. Stufe c) stammt.

Beispielsweise kann die bevorzugte Ausführungsform, wie die Figur es zeigt, durchgeführt werden: In einem Verdampfer (1) wird eine frisch zugeführte wäßrige Methanollösung (3) unter Einleiten von Luft (2) verdampft und das Dampfgemisch über die Verbindung (5) geleitet und im Reaktor (4) umgesetzt. Das Reaktionsgemisch wird über die Leitung (6) in die 1. Absorptionsstufe a) (Absorptionskolonne (7)) geführt, wo zusammen mit zugeführtem Harnstoff (21) die so gebildete Formaldehyd/Harnstoff-Lösung als Waschflüssigkeit dient und über die Verbindung (8) im Kreislauf geführt wird. Die konzentrierte Formaldehyd/Harnstoff-Lösung wird über Ausgangsleitung (9) abgezogen und weiterverarbeitet. Das Abgas tritt über die Verbindung (10) in die 2. Absorptionsstufe b) und von dort über Verbindung (24) in die 3. Absorptionsstufe, die zusammen in einer dreiteiligen Absorptionskolonne (14, 15, 16) untergebracht sind, ein. Im unteren Teil (14), der die 2. Absorptionsstufe b) (nur 1 Kolonnenteil) darstellt, wird die gebildete Absorptionslösung im Kreislauf (12) geführt und ein Teil der Lösung der 2. Stufe b) über die Ausgangsleitung (11) abgezogen und weiterverarbeitet. Gegebenenfalls wird über eine Zuführung (13) noch Wasser zugesetzt. Im mittleren Teil (15) wird die gebildete Absorptionslösung im Kreislauf (20) geführt. Verbindung (25) verbindet mittleren (15) mit oberem Teil (16). Im oberen Kolonnenteil (16) wird am Kopf (19) Wasser zugeführt, über die Leitung (23) ein Teil des Sumpfes im Kreislauf geführt und über den Ausgang (18) das Abgas entfernt. Über die Leitung (22) gelangt ein Sumpfanteil von (16) auf den Kopf von (15). Aus dem Sumpf des mittleren Kolonnenteils (15) wird kontinuierlich Formaldehydlösung entnommen und über die Verbindung (20) und die Verbindung (17) zum Verdampfer (1) zurückgeführt. Die Kolonnenteile (16) und (15) und die Verbindung (22) und (25) bilden die 3. Absorptionsstufe c).

Der nach dem Verfahren der Erfindung herstellbare Formaldehyd ist Desinfektionsmittel, Gerbstoff, Reduktionsmittel und wertvoller Ausgangsstoff für die Herstellung von Kunstharzen, Klebmitteln und Kunststoffen. Die erfindungsgemäßen Formaldehyd/Harnstoff-Lösungen sind wertvolle Ausgangsstoffe für die Herstellung von Kunstharzen, Klebmitteln und Kunststoffen. Bezüglich der Verwendung wird auf Ullmanns Encyklopädie der technischen Chemie, Band 7, Seite 670, und Band 3, Seiten 481 ff., verwiesen.

Die in dem folgenden Beispiel angeführten Teile bedeuten Gewichtsteile. Sie verhalten sich zu den Volumenteilen wie Kilogramm zu Liter. Die beiliegende Figur veranschaulicht eine der vorgenannten, bevorzugten Ausführungsformen, die gleichzeitig auch für das Beispiel Verwendung findet.

## Beispiel

Man verwendet eine Anlage mit Methanol-Verdampfer (1) und einem senkrechten Reaktor (4), wie sie in der vorgenannten Arbeitsweise (die Figur) veranschaulicht ist. Der Reaktor (4) enthält an seinem Kopf die Zuführung (5) für das dampfförmige Ausgangsgemisch und die Reaktorhaube. Die Katalysatorschicht liegt unterhalb des Reaktorkopfes, weiter unten folgt eine Kühlzone. Der Reaktor ist mit einer Absorptionsanlage, bestehend aus 4 Kolonnen (1. Absorptionsstufe a) = 1. Kolonne (7); 2. Absorptionsstufe b) = 2. Kolonne (14); 3. Absorptionsstufe c) = 3. Kolonne (15) und 4. Kolonne (16)), verbunden.

In den Reaktor (4) wird ein Katalysator aus Silberkristallen (0,187 Teilen) folgender Zusammensetzung eingetragen:

| | Anteil am Katalysator (Gew.-%) | Korngröße mm |
|---|---|---|
| Schicht 1 | 12,9 | 0,4–0,75 |
| Schicht 2 | 1,2 | 0,2–0,4 |
| Schicht 3 | 5,3 | 0,75–1 |
| Schicht 4 | 14,1 | 1–1,75 |
| Schicht 5 | 66,5 | 1–2,5 |

Schicht 2 wird als Ringschicht in der Randzone des Katalysators auf Schicht 3 aufgestreut. Der Durchmesser des Katalysators ist 170 cm, die lichte Weite der Ringschicht 167 cm. Der Reaktor ist über eine Leitung (6) mit der 1. Absorptionskolonne (7) verbunden. Dem Verdampfer (1) wird pro Stunde ein Gemisch von 4,78 Teilen Methanol, 0,01 Teilen Wasser und 8,14 Teilen Luft zugeführt und bei 92°C und 1,3 bar verdampft. Das Ausgangsgemisch wird durch den Katalysator (0,187 Teile) geleitet und bei 700°C und 1,3 bar umgesetzt. Das Reaktionsgemisch wird nun auf 150°C abgekühlt. Nun wird das dampfförmige Reaktionsgas über die Verbindung (6) durch die 1. Absorptionskolonne (7) von 5 Meter Länge (1. Stufe a)) und anschließend durch die 2. Absorptionsstufe b) (Kolonne 2) und 3. Absorptionsstufe c) (Kolonnen 3 und 4) geleitet. Kolonne 2 (14) hat eine Länge von 4 Metern, Kolonne 3 (15) und 4 (16) zusammen von 9 Metern. Die Kolonnen 1 bis 4 sind miteinander über die Verbindungen (10), (24), (25) verbunden und ihre Absorptionsflüssigkeiten werden jeweils im Kreislauf über die jeweiligen Kreislaufleitungen (8), (12), (20), (23) geführt. Ein Rücklauf von Flüssigkeit erfolgt zwischen dem Sumpf von Kolonne 4 (16) zum Kopf der Kolonne 3 (15) über die Verbindung (22), aber nicht zwischen Kolonnen 3 und 2 bzw. 2 und 1. Das Abgas wird durch die Kolonnen (7), (14), (15), (16) mittels der Verbindungen (10), (24), (25) geführt und tritt in (18) aus. Die Absorptionstemperaturen (gemessen am Kolonnenkopf) und pH-Werte sind in der 1. Kolonne 85°C, pH im Sumpf der Kolonne 6,4; in der 2. Kolonne 73°C, pH im Sumpf der Kolonne 4,5; in der 3. Kolonne 36°C, pH im Sumpf der Kolonne 4,6 und in der 4. Kolonne 31°C, pH im Sumpf der Kolonne 4,9. Der Durchsatz an Reaktionsgemisch (gemessen am Eintritt in die Kolonne) beträgt in der 1. Kolonne (1. Stufe) 3260, der Durchsatz an Abgas in der 2. Kolonne (2. Stufe) 2700, in der 3. Kolonne 2600 und in der

4. Kolonne 2400 kg pro Stunde und Quadratmeter Kolonnenquerschnitt. Kolonnen 1 (7), 2 (14) und 3 (15) sind Füllkörperkolonnen, Kolonne 4 (16) ist eine Glockenbodenkolonne. Über Einlaß (21) werden stündlich 0,99 Teile Harnstoff und 0,46 Teile Wasser und 0,002 Teile Natronlauge zugesetzt.

Die Absorptionsgemische enthalten:

1. Kolonne 55 Gew.-% Gesamtformaldehyd, 26,8 Gew.-% freien Formaldehyd, 22 Gew.-% Harnstoff, 26,5 Gew.-% Methylolharnstoffverbindungen (Mono- und Polymethylolharnstoffe), 0,8 Gew.-% Mono- und Polymethylenharnstoffe, 0,6 Gew.-% Polymethylolverbindungen von Methylenharnstoffen, 0,24 Gew.-% Methylenätherverbindungen, 0,15 Gew.-% höhermolekulare Kondensate, 0,5 Gew.-% Methanol, 0,06 Gew.-% Nebenstoffe (z. B. Ameisensäure).
2. Kolonne 40 Gew.-% Formaldehyd; 58,2 Gew.-% Wasser; 1,8 Gew.-% Methanol.
3. Kolonne 16,9 Gew.-% Formaldehyd; 80,3 Gew.-% Wasser; 2,8 Gew.-% Methanol.
4. Kolonne 3 Gew.-% Formaldehyd; 95,5 Gew.-% Wasser; 1,5 Gew.-% Methanol.

Pro Stunde werden dem Sumpf der 3. Kolonne (15) 3,6 Teile Formaldehydlösung (16,9 Gew.-% Formaldehyd; 80,3 Gew.-% Wasser; 2,8 Gew.-% Methanol) entnommen und über Leitung (17) zu dem Verdampfer rückgeführt. Pro Stunde werden in den Kreislauf der 4. Kolonne (16) über die Zuführung (19) 1,2 Teile Wasser zugegeben.

Die Belastung beträgt 2001 kg Methanol pro Quadratmeter Katalysatorbettquerschnitt und Stunde. Aus dem Sumpf der 1. (7) und 2. (14) Kolonne werden kontinuierlich 4,49 Teile des Endstoffes in Gestalt der konzentrierten Formaldehyd/Harnstofflösung über Ausgang (9) und 3,95 Teile der konzentrierten Formaldehydlösung über Ausgang (11) abgezogen. Die Formaldehydausbeute beträgt stündlich insgesamt 4,05 Teile, davon 2,47 Teile $CH_2O$ aus der 1. Stufe (7) und 1,58 Teile aus der 2. Stufe (14). Die als Endstoff anfallende Formaldehyd/Harnstoff-Lösung (stündlich 4,49 Teile) hat die folgende Zusammensetzung: 55 Gew.-% Gesamtformaldehyd, 26,8 Gew.-% freien Formaldehyd, 22 Gew.-% Harnstoff, 26,5 Gew.-% Methylolharnstoffverbindungen (Mono- und Polymethylolharnstoffe), 0,8 Gew.-% Mono- und Polymethylenharnstoffe, 0,6 Gew.-% Polymethylolverbindungen von Methylenharnstoffen, 0,24 Gew.-% Methylenätherverbindungen, 0,15 Gew.-% höhermolekulare Kondensate, 0,5 Gew.-% Methanol, 0,06 Gew.-% Nebenstoffe (darunter Ameisensäure). Man erhält stündlich durch Ausgang (11) 3,95 Teile 40gewichtsprozentige wäßrige Formaldehydlösung mit einem Methanolgehalt von 1,8 Gew.-% und einem Gehalt von 0,008 Gew.-% Ameisensäure. Das entspricht insgesamt einer Ausbeute an $CH_2O$ von 90,3% der Theorie und einem Umsatz von 95,6 Prozent. Die Ausbeute an Endstoff und der Methanol- und Ameisensäuregehalt der anfallenden Formaldehydlösung bleiben während 120 Tagen konstant. In der Katalysatorschicht treten keine Risse auf.

**Patentanspruch**

Verfahren zur gleichzeitigen Herstellung von konzentrierten, wäßrigen Formaldehyd/Harnstoff-Lösungen und Formaldehydlösungen durch oxidierende Dehydrierung von Methanol mit Luft in Gegenwart eines Silberkatalysators bei erhöhter Temperatur und anschließender Absorption des so gebildeten Formaldehyds in einer wäßrigen Lösung von Formaldehyd und Harnstoff bei einem pH unterhalb 7, dadurch gekennzeichnet, daß die Absorption

a) in einer ersten Stufe mit einer wäßrigen Lösung von 16 bis 30 Gewichtsprozent Harnstoff, von 20 bis 40 Gewichtsprozent freiem Formaldehyd und von 15 bis 40 Gewichtsprozent Methylolharnstoffverbindungen bei einem pH-Wert zwischen 2 und 7 und bei einer Temperatur von 80 bis 95° C,
b) dann in einer zweiten Stufe mit einer wäßrigen, 30- bis 45gewichtsprozentigen Formaldehydlösung bei einem pH-Wert von 2 bis 6 und bei einer Temperatur von 70 bis 85° C und
c) schließlich in einer dritten Stufe mit einer wäßrigen, 1- bis 30gewichtsprozentigen Formaldehydlösung bei einem pH-Wert von 2 bis 6 und bei einer Temperatur von 30 bis 50° C durchgeführt wird, und
d) aus der dritten Stufe kontinuierlich 0,1 bis 1,9 Mol Wasser und 0,04 bis 0,6 Mol Formaldehyd je Mol dem Katalysator insgesamt zugeführtem Methanol entnommen und dem Ausgangsgemisch der Methanoloxidation mit einer Belastung von 1000 bis 3000 kg Methanol je Quadratmeter Katalysatorbettquerschnitt und Stunde zugegeben werden.

**Claim**

A process for the simultaneous production of concentrated, aqueous formaldehyde/urea solutions and formaldehyde solutions by oxidizing dehydrogenation of methanol with air in the presence of a silver catalyst at elevated temperature, and subsequent absorption of the formaldehyde thus obtained in an aqueous solution of formaldehyde and urea at a pH below 7, characterized in that the absorption is carried out

(a) in a first stage with an aqueous solution of 16 to 30% by weight of urea, 20 to 40% by weight of free formaldehyde and 15 to 40% by weight of methylol urea compounds at a pH of from 2 to 7 and a temperature of from 80° to 95° C,

(b) then in a second stage with an aqueous, 30 to 45% strength by weight formaldehyde solution at a pH of from 2 to 6 and a temperature of from 70° to 85° C, and

(c) finally in a third stage with an aqueous, 1 to 30% strength by weight formaldehyde solution at a pH of from 2 to 6 and a temperature of from 30° to 50° C, and

(d) 0.1 to 1.9 moles of water and 0,04 to 0.6 mole of formaldehyde per mole of the total amount of methanol supplied to the catalyst are continuously removed from the third stage and added to the starting mixture of the methanol oxidation, the methanol being supplied at a rate of 1000 to 3000 kg per $m^2$ of catalyst bed cross-section per hour.

**Revendication**

Procédé pour la préparation simultanée de solutions aqueuses concentrées de formaldéhyde-urée et de formaldéhyde par une déshydrogénation oxydante du méthanol par l'air à température accrue en présence d'un catalyseur à l'argent, suivie de l'absorption du formaldéhyde formé dans une solution aqueuse de formaldéhyde et d'urée à un pH inférieur à 7, caractérisé en ce que l'on réalise l'absorption

a) dans un premier stade à une température de 80 à 95°C avec une solution aqueuse contenant 16 à 30% en poids d'urée, 20 à 40% en poids de formaldéhyde libre et 15 à 40% en poids de dérivés de méthylol-urée, d'un pH de 2 à 7,

b) dans un deuxième stade à une température de 70 à 85°C avec une solution aqueuse d'une concentration en formaldéhyde de 30 à 45% en poids et d'un pH de 2 à 6, et

c) dans un troisième stade à une température de 30 à 50°C avec une solution aqueuse d'une concentration en formaldéhyde de 1 à 30% en poids et d'un pH de 2 à 6, et

d) on soutire de façon continue du troisième stade, par mole de méthanol amenée au contact du catalyseur, 0,1 à 1,9 mole d'eau et 0,04 à 0,6 mole de formaldéhyde, que l'on recycle dans le mélange de départ pour l'oxydation du méthanol, avec une charge de 1000 à 3000 kg de méthanol par $m^2$ de section du lit de catalyseur et par heure.

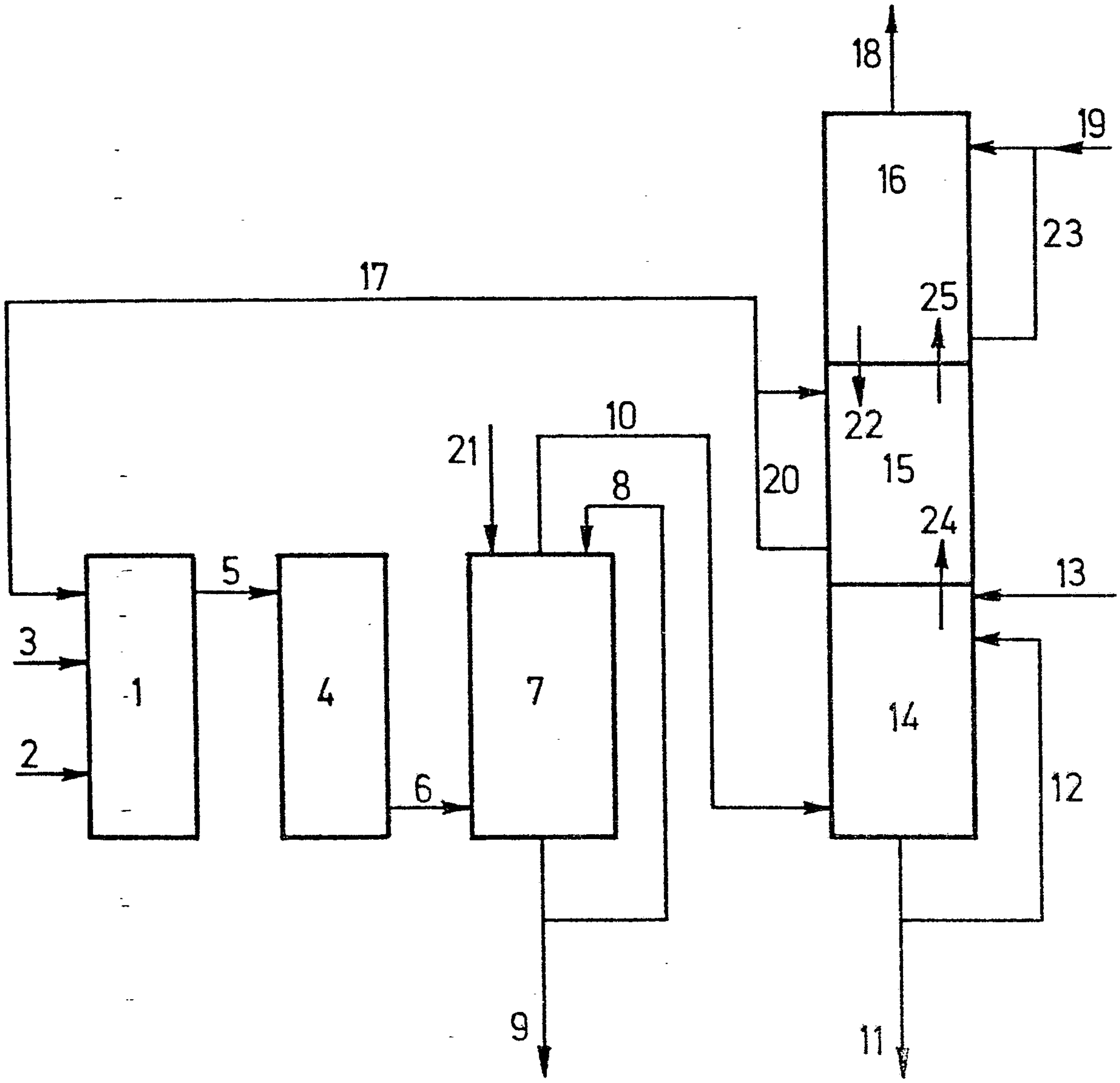
18
19
16
23
17
25
22
10
21
20
15
8
24
13
5
3
1
4
7
14
2
6
12
9
11